# EUROPEAN PATENT SPECIFICATION

(11) **EP 3 079 482 B1**
(45) Date of publication and mention of the grant of the patent: **31.01.2018**
(21) Application number: 14809619.1
(22) Date of filing: 03.12.2014
(51) Int. Cl.: A23D 7/005, C11B 5/00, A61K 8/36, A23L 33/11

(54) **EMULSIONS STABILIZED BY PARTICLES OF AN EDIBLE INORGANIC SALT**
EMULSIONEN DURCH PARTIKEL AUS EINEM ESSBAREN ANORGANISCHEN SALZ STABILISIERT
EMULSIONS STABILISÉES PAR DES PARTICULES D'UN SEL MINÉRAL COMESTIBLE

(30) Priority: 13.12.2013 EP 13197162
(43) Date of publication of application: 19.10.2016
(73) Proprietor: Nestec S.A., 1800 Vevey (CH)
(72) Inventor: GEHIN-DELVAL, Cécile, F-25370 Les Hôpitaux Neufs (FR); SCHMITT, Christophe Joseph Etienne, CH-1077 Servion (CH); BINKS, Bernard Paul, Walkington Yorkshire HU17 8XX (GB); DESTRIBATS, Mathieu Julien, F-33600 Pessac (FR)
(74) Representative: Lumsden, Stuart Edward Henry
(86) International application number: PCT/EP2014/076350
(87) International publication number: WO 2015/086388

(56) References cited:
- EP-A1- 1 736 532
- US-A- 4 208 432
- US-A- 5 366 661
- US-A1- 2011 206 824
- SZCZES ET AL: "Influence of the surfactant nature on the calcium carbonate synthesis in water-in-oil emulsion", JOURNAL OF CRYSTAL GROWTH, ELSEVIER, AMSTERDAM, NL, vol. 311, no. 4, 1 February 2009 (2009-02-01), pages 1129-1135, XP025991237, ISSN: 0022-0248, DOI: 10.1016/J.JCRYSGRO.2008.12.044 [retrieved on 2008-12-30]

## Description

The present invention relates to emulsions. In particular, it relates to an emulsion comprising at least one aqueous phase and at least one lipid phase wherein the emulsion is stabilized by particles of an edible inorganic salt having a solubility in water of less than 1 g per 100 ml at 20 °C and wherein the particles have between 0.5 and 20 wt. % fatty acids coated or adsorbed onto their surface. Further aspects of the invention are a composition comprising the emulsion and a method of manufacturing a double emulsion.

An emulsion consists of a mixture of two or more liquids that are normally immiscible. One or more liquid, the dispersed phase, is dispersed in the other, the continuous phase. In an oil-in-water emulsion for example, oil is the dispersed phase and water is the continuous phase. Emulsions are common in food, such as in mayonnaise, salad dressings, sauces, ice cream, and milk; and are frequently used in pharmaceutical, hairstyling, personal hygiene, and cosmetic products. Common emulsions are inherently unstable and thus do not form spontaneously. Energy input, such as shaking or mixing is needed to form an emulsion. Over time, emulsions tend to revert to the stable state of the phases comprising the emulsion. An example of this is seen in the separation of the oil and vinegar components of a simple vinaigrette salad dressing, an unstable emulsion that will quickly separate unless shaken almost continuously.

Emulsion stability is the ability of an emulsion to resist changes in its properties over time. Instability is mostly due to 3 phenomena: drainage, Ostwald ripening and coalescence. Drainage, or creaming, is where one of the substances migrates to the top of the emulsion due to buoyancy. Ostwald ripening is a thermodynamically-driven process due to the difference in osmotic pressure in droplets of different size. It results in the diffusion of molecules from the small droplets to the large ones through the continuous phase. Coalescence is the process in which two or more droplets merge during contact to form a single daughter droplet. Generally the three phenomena happen at the same time, leading to the instability of the emulsion and eventually the disappearance of the droplets and a return to a fully phase separated system.

Emulsifiers are substances which stabilize an emulsion by increasing its kinetic stability. One class of emulsifiers is known as "surface active substances", or surfactants. For example, adding mustard to a vinaigrette salad dressing can increase the stability of its emulsion, as chemicals in the mucilage surrounding the mustard seed hull act as emulsifiers. The emulsion will eventually disappear, but it remains longer than with oil and vinegar alone. There are a large number of emulsifiers available on the market, such as lecithins, proteins and low molecular weight emulsifiers.

Particles are also known to be able to stabilize emulsions. Particle-stabilized emulsions are sometimes known as Pickering emulsions [S.U. Pickering, J. Chem. Soc. Trans., 91, 2001 (1907)]. Much progress has been made in the last 10-15 years concerning the better understanding of particle-stabilized emulsions both from the theoretical and practical point of view. However, most of the particles used in these studies are synthetic, often inorganic materials that have limited applicability in food or pharmaceutical applications. Silica particles have been used to stabilize emulsions but they are generally partially hydrophobized (i.e. made hydrophobic to a partial extent) with adsorbed non-food grade, or even toxic, molecules or polymers. It would be desirable to develop other particles to stabilize emulsions which are of natural origin and are suitable for use in these applications. Emulsions have been stabilized by spores [B.P. Binks et al., Langmuir, 23, 9143 (2007)], chemically modified starch granules, cellulose particles and the water insoluble protein zein [J.W.J. de Folter et al., Soft Matter, 8, 2807 (2012)].

Double emulsions, or multiple emulsions, can be considered as an emulsion of an emulsion, where droplets of one dispersed liquid are further dispersed in another liquid. The major types of double emulsions are of water-in-oil-in-water (w/o/w) and oil-in-water-in-oil (o/w/o) double emulsions. In the present application, the drops of the encapsulated emulsion will be referred to as "droplets" and the drops of the encapsulating emulsion will be named "globules". For example in a water-in-oil-in-water emulsion, oil globules which are dispersed in an aqueous phase, contain themselves small water droplets.

Water-in-oil-in-water emulsions can be used to reduce the fat content of food products. For example WO2009/003960 describes how mayonnaise, which is traditionally an oil-in-water emulsion, can be reduced in fat by replacing the dispersed oil phase with a water-in-oil emulsion. The w/o/w emulsion is stabilized by a mixture of emulsifiers, with the external aqueous phase comprising at least one hydrophobic polymer or polymer aggregate.

The main challenge for formulating double emulsions is to achieve acceptable stability. Most solutions proposed to date rely on the proper choice of the emulsifiers used. For instance, EP0731685 describes a stable multiple emulsion obtained by using emulsifiers having a HLB value less than 6. EP0631774 also describes storage stable multiple emulsions comprising particular hydrophobic and hydrophilic emulsifiers. WO 03/049553 similarly relates to stable multiple emulsions obtained by selecting the appropriate emulsifiers used for the internal water-in-oil emulsion and for the external oil-in-water emulsion. US2003/0175317 describes double emulsions stabilized by particulate solids, for example a mixture of hydrophilic and hydrophobic silicon dioxide hydrophobicized by silylation. There is however still room for improvement. It would be advantageous to be able to produce stable formulations with food-grade emulsifiers and stabilizers instead of the synthetic surfactants and polymers that have been traditionally employed in most of the pharmaceutical and cosmetic applications. Many consumers are concerned about additives in food. It would be beneficial to provide emulsions stabilized by food grade materials which do not introduce a large number of extra ingredients to the ingredient list, especially ingredients with which the consumers are unfamiliar or consider to be artificial. In particular it would be beneficial to provide double emulsions stabilized by a small number of edible materials.

The object of the present invention is to improve the state of the art and to provide an improved solution to overcome at least some of the inconveniences described above or at least to provide a useful alternative. Any reference to prior art documents in this specification is not to be considered an admission that such prior art is widely known or forms part of the common general knowledge in the field. As used in this specification, the words "comprises", "comprising", and similar words, are not to be interpreted in an exclusive or exhaustive sense. In other words, they are intended to mean "including, but not limited to".

Accordingly, the present invention provides in a first aspect an edible emulsion comprising at least one aqueous phase and at least one lipid phase wherein the emulsion is stabilized by particles of an edible inorganic salt having a solubility in water of less than 1 g per 100 ml at 20 °C and wherein the particles have between 0.5 and 20 wt. % fatty acids coated or adsorbed onto their surface. In a second aspect, the invention relates to a composition comprising said emulsion. A third aspect of the invention is a method of manufacturing a double emulsion comprising the steps of; forming an aqueous dispersion of particles of an edible inorganic salt having between 0.5 and 20 wt. % of fatty acids coated or adsorbed onto their surface wherein the edible inorganic salt has a solubility in water of less than 1 g per 100 ml at 20 °C and at least 80 wt. % of the fatty acids are C6 to C12 fatty acids; forming an oil dispersion of particles of an edible inorganic salt having between 0.5 and 20 wt. % of fatty acids coated or adsorbed onto their surface wherein the edible inorganic salt has a solubility in water of less than 1 g per 100 ml at 20 °C and at least 80 wt. % of the fatty acids are C16 to C22 fatty acids; and either emulsifying an oil phase into said aqueous dispersion to form an oil-in-water emulsion and emulsifying the oil-in-water emulsion into said oil dispersion to form an oil-in-water-in-oil emulsion, or emulsifying an aqueous phase into said oil dispersion to form a water-in-oil emulsion and emulsifying the water-in-oil emulsion into said aqueous dispersion to form a water-in-oil-in-water emulsion.

The inventors initially tried to stabilize oil-in-water emulsions using uncoated particles of an edible inorganic salt, precipitated calcium carbonate. However, the obtained emulsions were not suitably stable under shear, their average drop diameters were not controlled and their polydispersity index was high. The inventors surprisingly found that by adsorbing fatty acids onto the particles, for example caprylic acid (C8:0), they were able to produce oil-in-water emulsions with good stability against shear, having droplets with a narrow size distribution and spherical shape. By using particles coated with a longer chain fatty acid such as stearic acid (C18:0), the inventors found they could stabilize water-in-oil emulsions.

The inventors investigated the formation of double emulsions. They were surprised to find that, rather than having to use a complex mixture of emulsifiers, they were able to form a double emulsion using particles having fatty acids coated or adsorbed onto their surface. For example, the inventors formed stable double emulsions by using calcium carbonate particles with caprylic acid adsorbed onto their surface to stabilize the oil-in-water interface, and calcium carbonate particles coated with stearic acid to stabilize the water-in-oil interface.

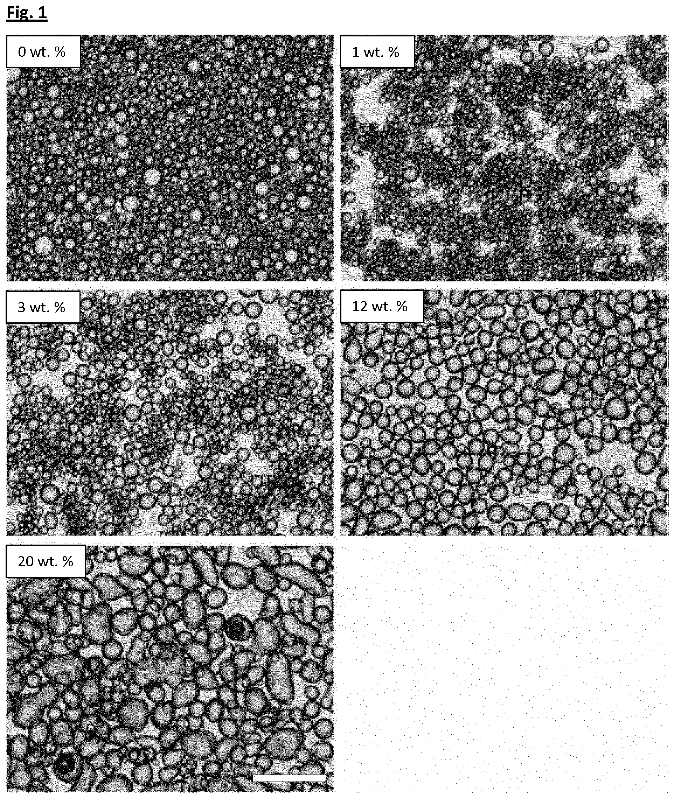

Figure 1 shows optical microscopy images of tricaprylin-in-water emulsions stabilized by a 2 wt. % dispersion of precipitated calcium carbonate particles functionalized with various wt. % of caprylic acid. Pictures have been taken one hour after emulsification and samples have been gently homogenised by hand shaking before observation. Scale bar is 500 µm.

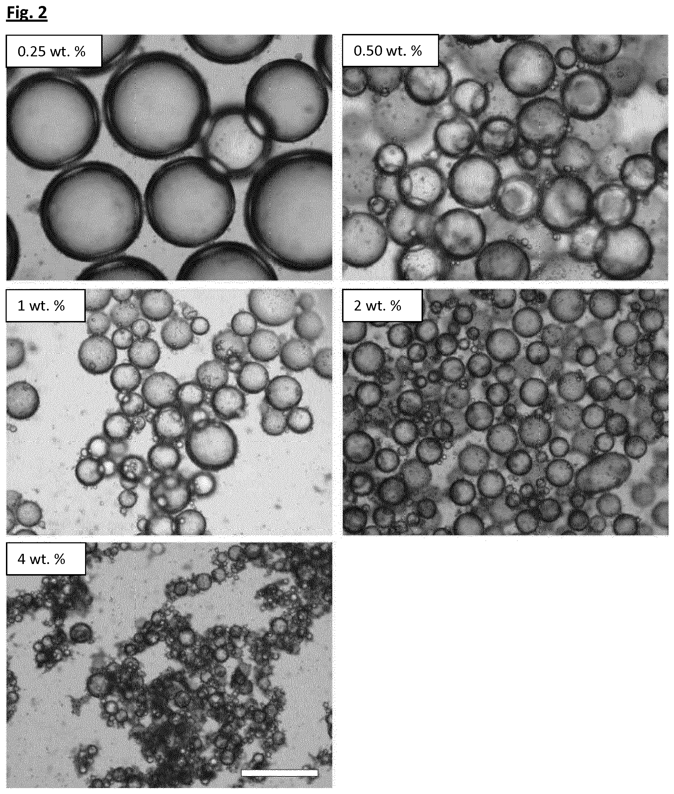

Figure 2 shows optical microscopy images of water-in-miglyol emulsions at various wt. % of precipitated calcium carbonate particles coated with 3 wt. % stearic acid. Pictures have been taken one hour after emulsification and samples have been gently homogenised by hand shaking before observation. Scale bar is 200 µm.

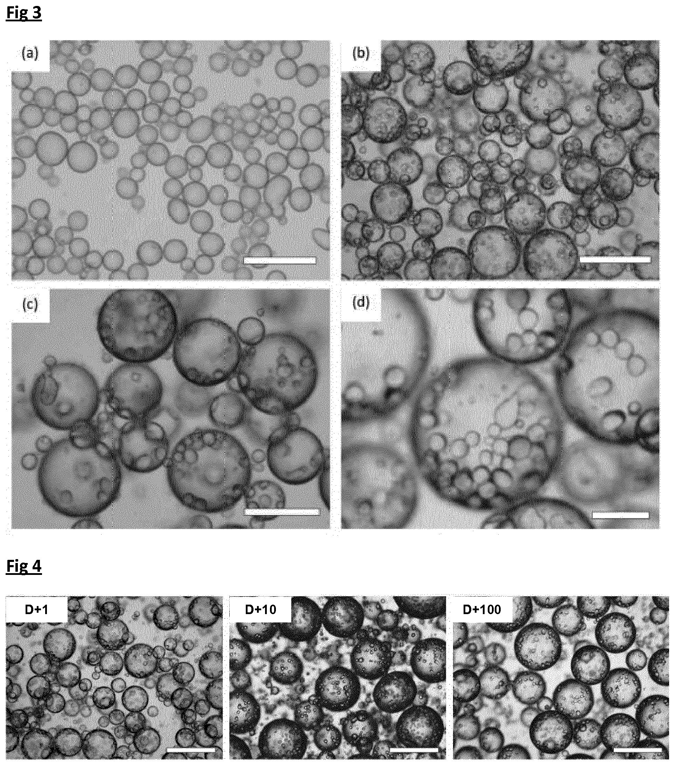

Figure 3 shows optical microscopy images of (a) emulsion 1, miglyol-in-water emulsion (10/90 vol. %) and (b), (c) and (d) emulsion 2, (miglyol-in-water)-in-miglyol emulsion at 20/80 vol. %. Pictures have been taken one hour after emulsification and samples have been gently homogenised by hand shaking before observation. Scale bars are 100 µm for (a) (c) (d) and 500 µm for (b).
Figure 4 shows optical microscopy images of emulsion 2, (miglyol-in-water)-in-miglyol emulsion at 20/80 vol. % after 1, 10 and 100 days. Samples have been gently homogenised by hand shaking before observation. Scale bars are 200 µm.

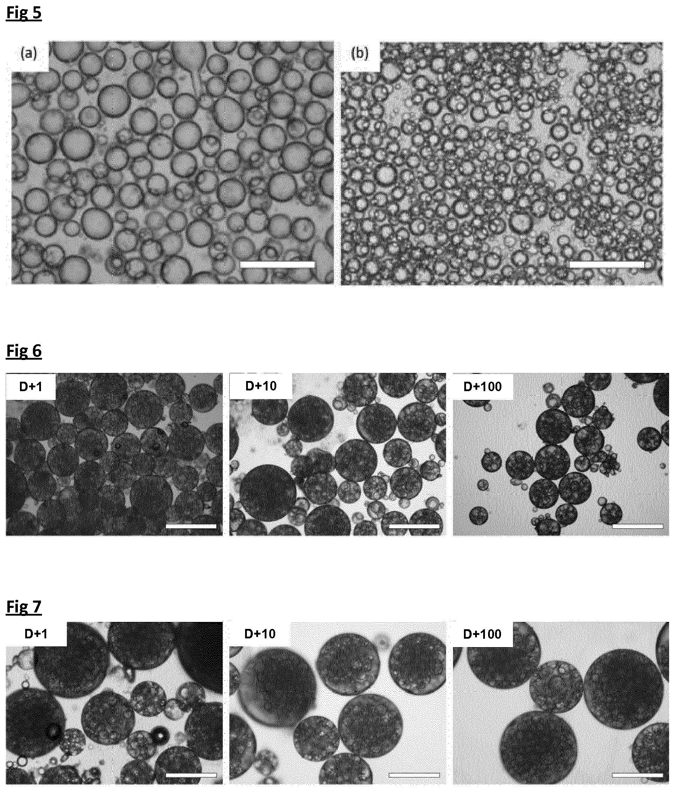

Figure 5 shows optical microscopy images of (a) emulsion 1, water-in-miglyol emulsion (20/80 vol%) and (b) emulsion 1, water-in-rapeseed oil emulsion (20/80 vol. %). Pictures have been taken one hour after emulsification and samples have been gently homogenised by hand shaking before observation. Scale bars are 200 µm.
Figure 6 shows optical microscopy images of emulsion 2, (water-in-miglyol)-in-water emulsion (20/80 vol. %) at 1, 10 and 100 days after emulsification. The samples have been gently homogenized by hand shaking before observation. Scale bars are 200 µm.
Figure 7 shows optical microscopy images of emulsion 2, (water-in- rapeseed oil)-in-water emulsion (20/80 vol. %) at 1, 10 and 100 days after emulsification. The samples have been gently homogenized by hand shaking before observation. Scale bars are 200 µm.

Consequently the present invention relates in part to an edible emulsion comprising at least one aqueous phase and at least one lipid phase wherein the emulsion is stabilized by particles of an edible inorganic salt having a solubility in water of less than 1 g per 100 ml at 20 °C and wherein the particles have between 0.5 and 20 wt. % fatty acids coated or adsorbed onto their surface. The emulsion may be stabilized by the particles of the edible inorganic salt being located at the interface(s) between the phases. The majority of the particles of the edible inorganic salt may be located at the interface(s) between the phases. The low solubility of the inorganic salt in water allows it to be used in aqueous environments without dissolving to an appreciable extent.

The term "edible" refers to substances which can be eaten safely. Whilst the current invention is not limited to substances permitted for consumption in any particular jurisdiction, edible emulsions may for example consist of materials approved for human consumption by the U.S. Food and Drug Administration. The term "aqueous phase" refers to a water-based portion of a system consisting of two or more phases while the "lipid phase" is the water-immiscible portion, typically oil-based. The aqueous phase may be water or an aqueous solution. The at least one lipid phase may consist of any non-polar substances which are not completely soluble in water. The at least one lipid phase may contain dissolved substances such as oil soluble compounds. The total lipid phase may be between 5 and 95 wt. % of the emulsion, for example between 15 and 85 wt. % of the emulsion. The total aqueous phase may be between 5 and 95 wt. % of the emulsion, for example between 15 and 85 wt. % of the emulsion.

The emulsion may be stabilized by particles of an edible inorganic salt having between 0.5 and 20 wt. % fatty acids coated or adsorbed onto their surface, for example between 3 and 12 wt. % fatty acids coated or adsorbed onto their surface.

The weight percentage of fatty acids is calculated based on the weight of uncoated particles.

Edible inorganic salt having a solubility in water of less than 1 g per 100 ml at 20 °C include calcium carbonate, calcium sulphate, magnesium carbonate, iron oxide, silicon dioxide and titanium dioxide. The emulsion of the invention may be stabilized by such edible particles. The inorganic salts may advantageously provide important dietary minerals when the emulsion is used in food, for example magnesium carbonate provides a dietary source of magnesium. It is advantageous that the emulsion is edible, even when the emulsion is not to be used as a food. For example, emulsions are used in cosmetic products such as sun-screens and may be inadvertently be ingested. Titanium dioxide is commonly used in sun screens so it is advantageous that it may also be used to stabilize emulsions.

Fatty acids are carboxylic acids with a long aliphatic tail (chain), which is either saturated or unsaturated. Most naturally occurring fatty acids have a chain of an even number of carbon atoms, from 4 to 28. Fatty acids are found in dietary fats, usually esterified with glycerol. It is an advantage to be able to stabilize an edible emulsion with ingredients which are familiar to consumers, and which may be obtained from natural sources. Fatty acids can be obtained from glycerol esters of fatty acids by, for example, hydrolysis. The term fatty acids in the context of the current invention means fatty acids as such, it does not include other molecules which have a fatty acid structure as part of the molecule, for example it does not include fatty acids esterified to a glycerol molecule such as in monoglycerides, diglycerides or triglycerides.

The edible inorganic salt in the emulsion of the invention may be calcium carbonate or calcium sulphate. Both calcium carbonate and calcium sulphate provide a dietary source of calcium. Calcium carbonate is a naturally occurring mineral. It is found in eggshells and seashells such as oyster shells as well as being present in dark green vegetables such as broccoli and kale. Calcium sulphate is also a naturally occurring mineral, for example in the form of gypsum.

The particles of the edible inorganic salt may be present at a level between 0.01 and 5 wt. % of the emulsion, for example between 1 and 3 wt. % of the emulsion. The weight percentage is calculated on the basis of uncoated particles as a percentage of the total emulsion.

The fatty acids coated or adsorbed onto the particles of the edible inorganic salt may be predominantly fatty acids with a chain length between 6 and 22 carbons. The fatty acids may be saturated or unsaturated aliphatic fatty acids. Short chain fatty acids generally have a strong odour which makes them difficult to use in food applications, whereas very long chain fatty acids have high melting points making them difficult to incorporate in food ingredients and impart a waxy mouth-feel. The fatty acids coated or adsorbed onto the particles may be mixtures of different fatty acids. The fatty acids may be selected from the group consisting of hexanoic acid, caprylic acid, capric acid, lauric acid, myristic acid, palmitic acid, stearic acid, arachidic acid, behenic acid and combinations of these. Preferably at least 80 wt. % of the fatty acids coated or adsorbed onto the particles have from 6 to 22 carbon atoms.

The edible emulsion of the invention may be a double emulsion. For example the emulsion may be a water-in-oil-in-water (w/o/w) or an oil-in-water-in-oil (o/w/o) emulsion. Generally, double emulsions require at least two different emulsifiers, one for the oil-in-water interface and one for the water-in-oil interface. In food grade double emulsion systems, the emulsion is usually stabilized by proteins and high levels of surfactants such as polyglycerol polyricinoleate. The interfacial adsorption of surfactant molecules is reversible, leading to limited stability of both the inner droplets and the outer globules. Gelling agents and viscosity increasing materials may be added to the aqueous and lipid phases to increase the stability of the double emulsion but despite this, double emulsion-based food products typically have short shelf lives. The particles of the edible inorganic salt used in the double emulsion of this invention adsorb irreversibly to both the oil-in-water and water-in-oil interfaces, leading to improved stability. In addition, many consumers do not like to see a long list of additives on the ingredient list of a food product, so for example it is an advantage to be able to stabilize a double emulsion with just particles of the edible inorganic salt and fatty acids, which have good acceptance as a food ingredients.

The particles of the edible inorganic salt may be hydrophobized to different extent by coating or adsorbing fatty acids onto their surface. The fatty acids may for example be coated onto an inorganic salt powder using a fluidized bed coating system. Fatty acids may for example be adsorbed onto the particles of the edible inorganic salt by forming an aqueous dispersion of inorganic salt particles together with fatty acids whilst applying ultrasound. Without wishing to be bound by theory, it is thought that, owing to their partial solubility in water and to the high energy input process, the fatty acids are able to reach the particle surface and remain adsorbed due to electrostatic interactions. Particles prepared in this way may also be described as particles "functionalized" by fatty acids. By employing particles hydrophobized to different extent it is possible to disperse the particles in either an aqueous phase (lower hydrophobicity particles) or a lipid phase (higher hydrophobicity particles). The particles hydrophobized to different extents can be used to stabilize oil-in-water and water-in-oil emulsions. For example an oil-in-water emulsion may be prepared by adding oil to a dispersion of lower hydrophobicity particles in water. The particles occupy the oil-in-water interface and hence stabilize the emulsion. Or, for example, a water-in-oil emulsion may be prepared by adding water to a dispersion of higher hydrophobicity particles in oil. The particles occupy the water-in-oil interface and hence stabilize the emulsion.

The inventors have found that particles of the edible inorganic salt hydrophobized to different extents are particularly beneficial in preparing double emulsions. The edible emulsion of the invention may use two types of particles of the edible inorganic salt; low hydrophobicity particles having fatty acids coated or adsorbed onto their surface such that at least 80 wt. % of the fatty acids are C6 to C12 fatty acids; and high hydrophobicity particles having fatty acids coated or adsorbed onto their surface such that at least 80 wt. % of the fatty acids are C16 to C22 fatty acids. For example the low hydrophobicity particles may have fatty acids coated or adsorbed onto their surface such that at least 80 wt. % of the fatty acids are C8 to C10 fatty acids; and high hydrophobicity particles may have fatty acids coated or adsorbed onto their surface such that at least 80 wt. % of the fatty acids are C18 to C20 fatty acids. For further example the low hydrophobicity particles may have fatty acids coated or adsorbed onto their surface such that at least 80 wt. % of the fatty acids are C8 fatty acids; and high hydrophobicity particles may have fatty acids coated or adsorbed onto their surface such that at least 80 wt. % of the fatty acids are C18 fatty acids.

The smaller fatty acids are more easily adsorbed onto the particles using the water dispersion technique as they have a greater solubility in water. Accordingly, the low hydrophobicity particles may have fatty acids adsorbed onto their surface such that at least 80 wt. % of the fatty acids are C6 to C12 fatty acids; and high hydrophobicity particles may have fatty acids coated onto their surface such that at least 80 wt. % of the fatty acids are C16 to C22 fatty acids. For example, the low hydrophobicity particles may have fatty acids adsorbed onto their surface such that at least 80 wt. % of the fatty acids are C8 to C10 fatty acids; and high hydrophobicity particles may have fatty acids coated onto their surface such that at least 80 wt.% of the fatty acids are C18 to C20 fatty acids. For further example, the low hydrophobicity particles may have fatty acids adsorbed onto their surface such that at least 80 wt. % of the fatty acids are C8 fatty acids; and high hydrophobicity particles may have fatty acids coated onto their surface such that at least 80 wt. % of the fatty acids are C18 fatty acids.

In an edible double emulsion of the invention, the majority of the low hydrophobicity particles may be at the oil-in-water interface and the majority of the high hydrophobicity particles may be at the water-in-oil interface. For example 60 wt. % of the fatty acids coated or adsorbed onto the particles at the oil-in-water interface may be C6 to C12 fatty acids; and 60 wt. % of the fatty acids coated or adsorbed onto the particles at the water-in-oil interface may be C16 to C22 fatty acids. For further example 60 wt. % of the fatty acids coated or adsorbed onto the particles at the oil-in-water interface may be C8 to C10 fatty acids; and 60 wt. % of the fatty acids coated or adsorbed onto the particles at the water-in-oil interface may be C18 to C20 fatty acids. For still further example, 60 wt. % of the fatty acids coated or adsorbed onto the particles at the oil-in-water interface may be C8 fatty acids; and 60 wt. % of the fatty acids coated or adsorbed onto the particles at the water-in-oil interface may be C18 fatty acids. The weight percent of the fatty acids being calculated on the basis of uncoated particles. In a water-in-oil-in-water double emulsion, the water-in-oil interface is between the droplets of aqueous phase surrounded by lipid phase in the encapsulated emulsion, and the oil-in-water interface is between the globules of encapsulated emulsion and the aqueous continuous phase. In an oil-in-water-in-oil double emulsion, the oil-in-water interface is between the droplets of lipid phase surrounded by aqueous phase in the encapsulated emulsion, and the water-in-oil interface is between the globules of encapsulated emulsion and the lipid continuous phase.

The at least one lipid phase of the emulsion of the invention may comprise oils selected from the group consisting of essential oils; sunflower oil; olive oil; palm oil; coconut oil, peanut oil; palm kernel oil; corn oil; hazelnut oil; sesame oil and mixtures of these. The essential oils may be the oil from a plant material selected from the group consisting of oregano, garlic, ginger, rose, mustard, cinnamon, rosemary, orange, grapefruit, lime, lemon, lemongrass, clove, clove leaf, vanilla, vanillin, mint, tea tree, thyme, grape seed, cilantro, lime, coriander, sage, eucalyptus, lavender, olive, olive leaf, anise, basil, pimento, dill, geranium, eucalyptus, aniseed, camphor, pine bark, onion, green tea, orange, artemisia herba-alba, aneth, citrus, marjoram, sage, ocimum gratissimum, thymus vulgaris, cymbopogon citratus, zingiber officinale, monodora myristica, and curcuma longa or a combination thereof. These oils are particularly suitable for use in food products, nutritional formulations or cosmetics.

The at least one lipid phase of the emulsion of the invention may contain oil soluble bioactive compounds. An oil-in-water emulsion or an oil-in-water-in-oil emulsion may advantageously be used to deliver oil soluble bioactive compounds dissolved in the oil droplets. By being contained within dispersed oil droplets the bioactive compounds may be better protected from degradation caused by processes such as oxidation. Within the scope of this invention, the term bioactive compound is understood to mean molecules or components showing biological activity or health impact when orally ingested or applied in cosmetics. The bioactive compounds may be selected from the group consisting of lipophilic carotenoids; polyphenols; vitamins, for example vitamins A and D; flavonoids; isoflavones; curcuminoids; ceramides; proanthocyanidins; terpenoids; caffeine, sterols; phytosterols; sterol-esters; tocotrienols; squalene; retinoids; and combinations thereof.

The edible emulsion of the invention may have a pH is between 3 and 8 when the edible inorganic salt is calcium carbonate, for example between 4 and 7. This pH range covers the values commonly encountered in foodstuffs and so it is an advantage to be able to stabilize emulsions in this pH range for culinary applications.

The particle stabilized emulsions of the invention have excellent stability against coalescence. The edible emulsion of the invention may be stable against coalescence for at least 10 days, for example they may be stable against coalescence for at least 50 days, for further example they may be stable against coalescence for at least 100 days. By "stable against coalescence" it is meant that the emulsions, stored motionless between 4 °C and 20 °C) do not exhibit an increase in average droplet diameter of more than 10 %.

The particles of the edible inorganic salt in the emulsion of the present invention may have an average diameter of less than 10 µm, for example less than 1 µm, for further example less than 100 nm. The term "average diameter" refers to the surface average diameter D[3;2]. The particles of the edible inorganic salt may be precipitated calcium carbonate. Precipitated calcium carbonate is generally a smaller particle size than other types of calcium carbonate such as ground calcium carbonate.

In a further aspect, the invention provides a composition comprising the emulsion of the invention. The composition may be a food composition; a beverage; a beverage enhancer, for example a coffee creamer; a cosmetic composition; a pharmaceutical composition; a nutritional formula; or a tube feeding formulation.

The emulsion of the invention may be used in a beverage. For example emulsions may be used to introduce hydrophobic nutritional ingredients into ready-to-drink beverages, the nutritional ingredient being present in a dispersed lipid phase. In soft drinks emulsions may provide colour, a desired cloudy appearance or flavour, for example a dispersion of flavour oils. The beverage may be selected from the group consisting of bottled water-based drinks, energy drinks, milk drinks and tea beverages.

Cosmetic products can benefit from the range of textures that may be obtained with the emulsions of the present invention, as well as the possibility of incorporating fat soluble bioactive materials within the oil droplets. In pharmaceutical compositions, double emulsions may be used to protect sensitive components, and to mask the unpleasant taste of some pharmaceutically active materials. The use of particles to stabilize an emulsion permits the formation of emulsions with a low viscosity continuous phase. This aids in the flow of the emulsion and so is an advantage for tube feeding compositions. By incorporating active ingredients in the inner aqueous phase of o/w/o double emulsions a targeted release in the gastrointestinal system may be achieved. The composition comprising the emulsion of the invention may be a nutritional formula. The nutritional formula may be a complete nutritional formula which provides sufficient types and levels of macronutrients (protein, fats and carbohydrates) and micronutrients to be sufficient as a sole source of nutrition for the subject to which it is administered. The nutritional formula may also provide partial nutrition, to act as a supplement to the existing diet of the subject.

The stability of the emulsion of the invention, and the edible nature of the components makes it ideal for use in foods, for example emulsions such as mayonnaise, low-fat spreads, vinaigrette, ice-cream, sauces and soups. Particles of the edible inorganic salt and fatty acids are also considered more acceptable to consumers than the apparently synthetic chemical names of many commercial food emulsifiers, especially those used to stabilize double emulsions. Double emulsions have a number of valuable applications in foods. They may be used to encapsulate or protect sensitive and active food components from the environment, for example to protect against oxidation such as in an omega-3-enriched tuna spread, being an o/w/o emulsion with the omega-3 oils in the inner oil droplets. Double emulsions may be used to control aroma and flavour release or to produce foods with a lower oil or fat content. The food composition comprising the emulsion of the invention may be a dairy product; an ice-cream; a sauce; a soup; a spread; a dessert; a confectionery product; a bakery product; a salad dressing; or a pet food.

Sodium, found in common salt (sodium chloride), is important for controlling the amount of water in the body, maintaining the normal pH of blood, transmitting nerve signals and helping muscular contraction. Salt is present in foods in different amounts, but consuming too much can have adverse effects on health. The World Health Organization recommends adults consume no more than about one teaspoon of salt per person per day. Globally, many governments and public health bodies have launched initiatives to help people reduce their salt intake. A double emulsion according to the invention may be used to enhance saltiness perception in a food product. For example, in a soup which is an oil-in-water emulsion seasoned with sodium chloride, the sodium chloride is mainly dissolved in the continuous aqueous phase. By including some of the water as droplets within the dispersed oil phase, in other words creating a w/o/w emulsion, and having the sodium chloride only in the continuous aqueous phase and not in the aqueous droplets, the concentration of sodium chloride in the continuous phase can be maintained, while the overall concentration of sodium chloride in the soup is reduced. This may allow the saltiness perception to be maintained for a lower total sodium chloride content of the soup. Double emulsions according to the invention, stabilized by particles of an edible inorganic salt, are particularly suitable for enhancing saltiness perception in this way as the effectiveness of the particles as emulsion stabilizers is not affected by the ionic strength of the aqueous phase. This allows locally high concentrations of salt to be maintained. The food composition comprising the emulsion of the invention may contain 140 mg of sodium or less per 100 g. The U.S. Food and Drug Administration define meals and main dishes to be "low in sodium" if they contain 140 mg or less of sodium per 100g. The U.S. Food and Drug Administration define meals and main dishes to be "very low in sodium" if they contain 35 mg or less of sodium per 100 g. The food composition comprising the emulsion of the invention may contain 35 mg of sodium or less per 100 g.

An edible oil-in-water emulsion according to the invention may be manufactured by the steps of forming an aqueous dispersion of particles of an edible inorganic salt having between 0.5 and 20 wt. % of fatty acids coated or adsorbed onto their surface wherein the edible inorganic salt has a solubility in water of less than 1 g per 100 ml at 20 °C and wherein at least 80 wt. % of the fatty acids are C6 to C12 fatty acids; and emulsifying an oil phase into said aqueous dispersion. Particles having shorter chain length fatty acids adsorbed or coated onto their surface may advantageously be used to form an aqueous dispersion as they have some affinity for water. They are then able to act as emulsifiers and stabilize oil droplets within the aqueous dispersion. As discussed above, fatty acids may be adsorbed onto the particles by forming an aqueous dispersion of the particles together with fatty acids whilst applying ultrasound. Conveniently, this directly leads to an aqueous dispersion of particles having fatty acids adsorbed on their surface which can be used to emulsify oil and so form an oil-in-water emulsion. It is also possible to coat fatty acids onto the edible inorganic salt, such as in a fluid-bed coater, but this may be less efficient as it requires an extra piece of equipment (the coating apparatus). Accordingly, an edible oil-in-water emulsion according to the invention may be manufactured by the steps of forming an aqueous dispersion of particles of an edible inorganic salt together with fatty acids whilst applying ultrasound, such that the particles have between 0.5 and 20 wt. % of fatty acids adsorbed onto their surface, wherein the edible inorganic salt has a solubility in water of less than 1 g per 100 ml at 20 °C, and and wherein at least 80 wt. % of the fatty acids are C6 to C12 fatty acids; and emulsifying an oil phase into said aqueous dispersion.

An edible water-in-oil emulsion according to the invention may be manufactured by the steps of forming an oil dispersion of edible inorganic salt particles having between 0.5 and 20 wt. % of fatty acids coated or adsorbed onto their surface, wherein the edible inorganic salt has a solubility in water of less than 1 g per 100 ml at 20 °C, and wherein at least 80 wt. % of the fatty acids are C16 to C22 fatty acids; and emulsifying an aqueous phase into said oil dispersion.

In a further aspect, the invention provides a method of manufacturing a double emulsion comprising the steps of forming an aqueous dispersion of particles of an edible inorganic salt having between 0.5 and 20 wt. % of fatty acids (for example between 2 and 12 wt. % of fatty acids) coated or adsorbed onto their surface wherein the edible inorganic salt has a solubility in water of less than 1 g per 100 ml at 20 °C and wherein at least 80 wt. % of the fatty acids are C6 to C12 fatty acids (for example at least 80 wt. % of the fatty acids are C8 to C10 fatty acids); forming an oil dispersion of particles of an edible inorganic salt having between 0.5 and 20 wt. % of fatty acids (for example between 2 and 12 wt. % of fatty acids) coated or adsorbed onto their surface wherein the edible inorganic salt has a solubility in water of less than 1 g per 100 ml at 20 °C and wherein at least 80 wt. % of the fatty acids are C16 to C22 fatty acids (for example at least 80 wt. % of the fatty acids are C18 to C20 fatty acids); and either emulsifying an oil phase into said aqueous dispersion to form an oil-in-water emulsion and emulsifying the oil-in-water emulsion into said oil dispersion to form an oil-in-water-in-oil emulsion; or emulsifying an aqueous phase into said oil dispersion to form a water-in-oil emulsion and emulsifying the water-in-oil emulsion into said aqueous dispersion to form a water-in-oil-in-water emulsion.

As discussed above, fatty acids may be adsorbed onto particles of an edible inorganic salt by forming an aqueous dispersion of particles together with fatty acids whilst applying ultrasound, which conveniently directly leads to an aqueous dispersion of particles having fatty acids adsorbed on their surface. Accordingly, the method of manufacturing a double emulsion of the invention may comprise the steps of forming an aqueous dispersion of particles of an edible inorganic salt having between 0.5 and 20 wt. % of fatty acids (for example between 2 and 12 wt. % of fatty acids) adsorbed onto their surface wherein the edible inorganic salt has a solubility in water of less than 1 g per 100 ml at 20 °C and wherein at least 80 wt. % of the fatty acids are C6 to C12 fatty acids (for example at least 80 wt. % of the fatty acids are C8 to C10 fatty acids); forming an oil dispersion of particles of an edible inorganic salt having between 0.5 and 20 wt. % of fatty acids (for example between 2 and 12 wt. % of fatty acids) coated or adsorbed onto their surface wherein the edible inorganic salt has a solubility in water of less than 1 g per 100 ml at 20 °C and wherein at least 80 wt. % of the fatty acids are C16 to C22 fatty acids (for example at least 80 wt. % of the fatty acids are C18 to C20 fatty acids); and either emulsifying an oil phase into said aqueous dispersion to form an oil-in-water emulsion and emulsifying the oil-in-water emulsion into said oil dispersion to form an oil-in-water-in-oil emulsion; or emulsifying an aqueous phase into said oil dispersion to form a water-in-oil emulsion and emulsifying the water-in-oil emulsion into said aqueous dispersion to form a water-in-oil-in-water emulsion.

Those skilled in the art will understand that they can freely combine all features of the present invention disclosed herein. In particular, features described for the product of the present invention may be combined with the method of the present invention and vice versa. Further, features described for different embodiments of the present invention may be combined. Where known equivalents exist to specific features, such equivalents are incorporated as if specifically referred to in this specification. Further advantages and features of the present invention are apparent from the figures and non-limiting examples.

### Examples

### Example 1: Oil-in-water emulsion

An aqueous dispersion was formed by dispersing uncoated precipitated calcium carbonate (CALOFORT™ U, Speciality Minerals) in 10 mL water at a level of 5 wt. % with between 0-20 wt. % of caprylic acid with respect of the particle mass. The dispersion was promoted by applying ultrasound with a Branson digital sonicator fitted with an ultrasound probe (6 mm, 30% amplitude, 1 s pulse on, 0.5 s pulse off) operating for 5 min. The caprylic acid becomes adsorbed on the particle surface due to electrostatic interactions.

Tricaprylin oil (Sigma) was then emulsified into the aqueous dispersion using an Ultra-Turrax IKA T25 mixer equipped with a small dimension head (UT SH), at constant speed (13500 rpm) for 30s. Figure 1 shows the tricaprylin-in-water (50/50 vol.) emulsions stabilized by 2 wt. % calcium carbonate dispersions for different amounts of caprylic acid adsorbed onto the calcium carbonate. The emulsion with uncoated calcium carbonate particles (0 wt. % caprylic acid) was not suitably stable (destabilized under shear), the average drop diameter was not controlled and the polydispersity index was high. The emulsions with the better properties in term of stability against shear, control of the drop size, narrow size distribution and spherical shape were those stabilized by particles that had been modified within a 3 to 12 wt. % range of caprylic acid.

### Example 2: Water-in-oil emulsion

Precipitated calcium carbonate particles coated with 3% stearic acid (CALOFORT™ SV, Speciality Minerals) were dispersed at various concentrations in a mixture of C8:0 and C10:0 fatty acids (Miglyol 812, Sasol) using an ultrasound probe (3 mm, 80 % amp., 5 min). Then water-in-miglyol emulsions (20/80 vol. %) were prepared using an Ultra-Turrax mixer equipped with a large dimension head (UT LH), at constant speed (16000 rpm) for 30s. Figure 2 shows emulsions stabilized by various amounts of particles and, following a limited coalescence phenomenon, the emulsion diameters range from 260 to 10 µm.

### Example 3: Double emulsions

Double emulsions were prepared in two steps. At first, a high-shear homogenization (UT LH operating at 16000 rpm for 30 s) was applied to produce emulsion 1. In a second step, emulsion 1 is added to the final continuous phase and the system is gently manually shaken for 30 s to produce emulsion 2. In the following, three different kinds of double emulsions are presented varying the emulsion type (o/w/o or w/o/w) and the oil type (Miglyol or rapeseed oil (Tesco)), but using only two types of calcium carbonate particles; precipitated calcium carbonate with 6 wt. % of caprylic acid adsorbed onto it, and precipitated calcium carbonate coated with 3 wt. % stearic acid.

### Oil-in-water-in-oil

Emulsion 1: miglyol-in-water emulsion (10/90 vol. %) stabilized by a 0.55 wt. % of precipitated calcium carbonate with 6 wt. % adsorbed caprylic acid. The average droplet diameter was around 30 µm. See Figure 3a.
Emulsion 2: (miglyol-in-water)-in-miglyol emulsion at 20/80 vol. %. Emulsion 2 is stabilized by a 0.31 wt. % dispersion in miglyol of precipitated calcium carbonate coated with 3 wt. % stearic acid. See Figure 3 (b, c and d).

After 10 days, the double emulsion globules looked slightly bigger, suggesting they experienced some coalescence events; nevertheless the inner droplets seemed stable. See Figure 4.

### Water-in-oil-in-water (oil = Miglyol)

Emulsion 1: water-in-miglyol emulsion (20/80 vol. %) stabilized by a 2 wt. % dispersion of precipitated calcium carbonate coated with 3 wt. % stearic acid. Average droplet diameter around 30-50 µm. See Figure 5(a).
Emulsion 2: (water-in-miglyol)-in-water emulsion at 20/80 vol. %. Emulsion 2 is stabilized by a 0.25 wt. % aqueous dispersion of precipitated calcium carbonate with 6 wt. % adsorbed caprylic acid. See Figure 6.

### Water-in-oil-in-water (oil = Rapeseed oil)

Emulsion 1: water-in-rapeseed oil emulsion (20/80 vol. %) stabilized by a 2 wt. % dispersion of precipitated calcium carbonate coated with 3 wt. % stearic acid. Average droplet diameter around 20 µm. See Figure 5(b).
Emulsion 2: (water-in-rapeseed oil)-in-water emulsion at 20/80 vol. %. Emulsion 2 is stabilized by a 0.25 wt. % aqueous dispersion of precipitated calcium carbonate with 6 wt. % adsorbed caprylic acid, see figure 7. The double emulsion is still stable after 100 days, see Figure 7(d).

## Claims

1. Edible emulsion comprising at least one aqueous phase and at least one lipid phase wherein the emulsion is stabilized by particles of an edible inorganic salt having a solubility in water of less than 1 g per 100 ml at 20 °C and wherein the particles have between 0.5 and 20 wt. % fatty acids coated or adsorbed onto their surface.

2. An edible emulsion according to claim 1 wherein the edible inorganic salt is calcium carbonate or calcium sulphate.

3. An edible emulsion according to claim 1 or claim 2 wherein the particles of the edible inorganic salt are present at a level between 0.01 and 5 wt. % of the emulsion.

4. An edible emulsion according to any one of claims 1 to 3 wherein at least 80 wt. % of the fatty acids coated or adsorbed onto the particles of the edible inorganic salt have from 6 to 22 carbon atoms.

5. An edible emulsion according to any one of claims 1 to 4 wherein the emulsion is a double emulsion.

6. An edible emulsion according to claim 5 wherein two types of particles of the edible inorganic salt are used; low hydrophobicity particles having fatty acids coated or adsorbed onto their surface such that at least 80 wt. % of the fatty acids are C6 to C12 fatty acids; and high hydrophobicity particles having fatty acids coated or adsorbed onto their surface such that at least 80 wt. % of the fatty acids are C16 to C22 fatty acids.

7. An edible emulsion according to claim 6 wherein the majority of the low hydrophobicity particles are at the oil-in-water interface and the majority of the high hydrophobicity particles are at the water-in-oil interface.

8. An edible emulsion according to any one of claims 1 to 7 wherein the at least one lipid phase comprises oils selected from the group consisting of essential oils; sunflower oil; olive oil; palm oil; coconut oil, peanut oil; palm kernel oil; corn oil; hazelnut oil; sesame oil and mixtures of these.

9. An edible emulsion according to any one of claims 1 to 8 wherein the emulsion is stable against coalescence for at least 10 days.

10. An edible emulsion according to any one of claims 1 to 9 wherein the particles of the edible inorganic salt have an average diameter of less than 10µm.

11. An edible emulsion according to any one of claims 1 to 10 wherein the particles of the edible inorganic salt are precipitated calcium carbonate.

12. Composition comprising the emulsion of any one of claims 1 to 11 wherein the composition is a food composition; a beverage; a beverage enhancer; a cosmetic composition; a pharmaceutical composition; a nutritional formula; or a tube feeding formulation.

13. A composition according to claim 12 wherein the food composition is a dairy product; an ice-cream; a sauce; a soup; a spread; a dessert; a confectionery product; a bakery product; a salad dressing; or a pet food.

14. A composition according to claim 12 or claim 13 wherein the food composition contains 140 mg of sodium or less per 100 g.

15. Method of manufacturing a double emulsion comprising the steps of
- forming an aqueous dispersion of particles of an edible inorganic salt having between 0.5 and 20 wt. % of fatty acids coated or adsorbed onto their surface wherein the edible inorganic salt has a solubility in water of less than 1 g per 100 ml at 20 °C and wherein at least 80 wt. % of the fatty acids are C6 to C12 fatty acids
- forming an oil dispersion of particles of an edible inorganic salt having between 0.5 and 20 wt. % of fatty acids coated or adsorbed onto their surface wherein the edible inorganic salt has a solubility in water of less than 1 g per 100 ml at 20 °C and wherein at least 80 wt. % of the fatty acids are C16 to C22 fatty acids
- and either
emulsifying an oil phase into said aqueous dispersion to form an oil-in-water emulsion and emulsifying the oil-in-water emulsion into said oil dispersion to form an oil-in-water-in-oil emulsion,
or
emulsifying an aqueous phase into said oil dispersion to form a water-in-oil emulsion and emulsifying the water-in-oil emulsion into said aqueous dispersion to form a water-in-oil-in-water emulsion.

## Patentansprüche

1. Essbare Emulsion, umfassend mindestens eine wässrige Phase und mindestens eine Lipidphase, wobei die Emulsion durch Teilchen eines essbaren anorganischen Salzes mit einer Löslichkeit in Wasser von weniger als 1 g pro 100 ml bei 20 °C stabilisiert wird, und wobei die Teilchen auf deren Oberfläche zu zwischen 0,5 und 20 Gew.-% mit Fettsäuren beschichtet sind oder diese adsorbiert haben.

2. Essbare Emulsion nach Anspruch 1, wobei das essbare anorganische Salz Calciumcarbonat oder Calciumsulfat ist.

3. Essbare Emulsion nach Anspruch 1 oder Anspruch 2, wobei die Teilchen des essbaren anorganischen Salzes in einer Höhe von zwischen 0,01 und 5 Gew.-% der Emulsion vorliegen.

4. Essbare Emulsion nach einem der Ansprüche 1 bis 3, wobei mindestens 80 Gew.-% der Fettsäuren, mit denen die Teilchen des essbaren anorganischen Salzes beschichtet oder die darauf adsorbiert wurden, von 6 bis 22 Kohlenstoffatome aufweisen.

5. Essbare Emulsion nach einem der Ansprüche 1 bis 4, wobei die Emulsion eine Doppelemulsion ist.

6. Essbare Emulsion nach Anspruch 5, wobei die beiden Arten von Teilchen des essbaren anorganischen Salzes verwendet werden; Teilchen mit geringer Hydrophobizität, deren Oberflächen mit Fettsäuren beschichtet oder darauf adsorbiert wurden, sodass mindestens 80 Gew.-% der Fettsäuren C6- bis C12-Fettsäuren sind; und Teilchen mit hoher Hydrophobizität mit Fettsäuren, deren Oberflächen mit Fettsäuren beschichtet oder darauf adsorbiert wurden, sodass mindestens 80 Gew.-% der Fettsäuren C16- bis C22-Fettsäuren sind.

7. Essbare Emulsion nach Anspruch 6, wobei sich die Mehrheit der Teilchen mit geringer Hydrophobizität an der Öl-Wasser-Grenzfläche befindet und sich die Mehrheit der Teilchen mit hoher Hydrophobizität an der Wasser-Öl-Grenzfläche befindet.

8. Essbare Emulsion nach einem der Ansprüche 1 bis 7, wobei die mindestens eine Lipidphase Öle umfasst, die ausgewählt sind aus der Gruppe bestehend essenziellen Ölen; Sonnenblumenöl; Olivenöl; Palmöl; Kokosöl, Erdnussöl; Palmkernöl; Maisöl; Haselnussöl; Sesamöl und Mischungen von diesen.

9. Essbare Emulsion nach einem der Ansprüche 1 bis 8, wobei die Emulsion mindestens 10 Tage lang koaleszenzstabil ist.

10. Essbare Emulsion nach einem der Ansprüche 1 bis 9, wobei die Teilchen des essbaren anorganischen Salzes einen durchschnittlichen Durchmesser von weniger als 10 µm aufweisen.

11. Essbare Emulsion nach einem der Ansprüche 1 bis 10, wobei es sich bei den Teilchen des essbaren anorganischen Salzes um gefälltes Calciumcarbonat handelt.

12. Zusammensetzung, umfassend die Emulsion nach einem der vorstehenden Ansprüche 1 bis 11, wobei die Zusammensetzung eine Nahrungsmittelzusammensetzung; ein Getränk; ein Getränkeverbesserer; eine kosmetische Zusammensetzung; eine pharmazeutische Zusammensetzung; eine Ernährungsformulierung; oder eine Sondenernährungsformulierung ist.

13. Zusammensetzung nach Anspruch 12, wobei die Nahrungsmittelzusammensetzung ein Milchprodukt; eine Eiscreme; eine Soße; eine Suppe; ein Aufstrich; eine Nachspeise; ein Süßwarenprodukt; eine Backware; ein Salatdressing oder ein Haustierfutter ist.

14. Zusammensetzung nach Anspruch 12 oder Anspruch 13, wobei die Nahrungsmittelzusammensetzung 140 mg Natrium oder weniger pro 100 g enthält.

15. Verfahren zur Herstellung einer Doppelemulsion, umfassend die Schritte
- Bilden einer wässrigen Dispersion aus Teilchen eines essbaren anorganischen Salzes, deren Oberflächen mit zwischen 0,5 und 20 Gew.-% Fettsäuren beschichtet oder darauf adsorbiert sind, wobei das essbare anorganische Salz eine Löslichkeit in Wasser von weniger als 1 g pro 100 ml bei 20 °C aufweist, und wobei mindestens 80 Gew.-% der Fettsäuren C6- bis C12-Fettsäuren sind,
- Bilden einer Öldispersion aus Teilchen eines essbaren anorganischen Salzes, deren Oberflächen zu zwischen 0,5 und 20 Gew.-% mit Fettsäuren beschichtet oder darauf adsorbiert sind, wobei das essbare anorganische Salz eine Löslichkeit in Wasser von weniger als 1 g pro 100 ml bei 20 °C aufweist, und wobei mindestens 80 Gew.-% der Fettsäuren C16- bis C22-Fettsäuren sind,
- und entweder
Emulgieren einer Ölphase in die wässrige Dispersion zur Bildung einer Öl-in-Wasser-Emulsion und Emulgieren der Öl-in-Wasser-Emulsion in die Öldispersion zur Bildung einer Öl-in-Wasser-in-Öl-Emulsion,
oder
Emulgieren einer wässrigen Phase in die Öldispersion zur Bildung einer Wasser-in-Öl-Emulsion und Emulgieren der Wasser-in-Öl-Emulsion in die wässrige Dispersion zur Bildung einer Wasser-in-Öl-in-Wasser-Emulsion.

## Revendications

1. Émulsion comestible comprenant au moins une phase aqueuse et au moins une phase lipidique, où laquelle l'émulsion est stabilisée par des particules d'un sel inorganique comestible présentant une solubilité dans l'eau inférieure à 1 g par 100 ml à 20 °C et dans laquelle les particules comportent des acides gras revêtus ou adsorbés sur leur surface dans une proportion comprise entre 0,5 % en poids et 20 % en poids.

2. Émulsion comestible selon la revendication 1, dans laquelle le sel inorganique comestible est du carbonate de calcium ou du sulfate de calcium.

3. Émulsion comestible selon la revendication 1 ou la revendication 2, dans laquelle les particules du sel inorganique comestible sont présentes à un taux compris entre 0,01 et 5 % en poids de l'émulsion.

4. Émulsion comestible selon l'une quelconque des revendications 1 à 3, dans laquelle au moins 80 % en poids des acides gras revêtus ou adsorbés sur les particules du sel inorganique comestible possèdent de 6 à 22 atomes de carbone.

5. Émulsion comestible selon l'une quelconque des revendications 1 à 4, où l'émulsion est une émulsion double.

6. Émulsion comestible selon la revendication 5, dans laquelle on utilise deux types de particules du sel inorganique comestible ; des particules à faible hydrophobicité comportant des acides gras formant un revêtement ou adsorbés sur leur surface de sorte qu'au moins 80 % en poids des acides gras sont des acides gras en C6 à C12 ; et des particules à faible hydrophobicité comportant des acides gras formant un revêtement ou adsorbés sur leur surface de sorte qu'au moins 80 % en poids des acides gras sont des acides gras en C16 à C22.

7. Émulsion comestible selon la revendication 6, dans laquelle la majorité des particules à faible hydrophobicité se trouvent à l'interface huile-dans-l'eau et la majorité des particules à haute hydrophobicité se trouvent à l'interface eau-dans-l'huile.

8. Émulsion comestible selon l'une quelconque des revendications 1 à 7, dans laquelle la ou les phases lipidiques comprennent des huiles choisies dans le groupe composé d'huiles essentielles ; de l'huile de tournesol ; de l'huile d'olive ; de l'huile de palme ; de l'huile de noix de coco, de l'huile d'arachide ; de l'huile de palmiste ; de l'huile de maïs ; de l'huile de noisette ; de l'huile de sésame et de leurs mélanges.

9. Émulsion comestible selon l'une quelconque des revendications 1 à 8, où l'émulsion est stable vis-à-vis de la coalescence pendant au moins 10 jours.

10. Émulsion comestible selon l'une quelconque des revendications 1 à 9, dans laquelle les particules du sel inorganique comestible ont un diamètre moyen inférieur à 10 µm.

11. Émulsion comestible selon l'une quelconque des revendications 1 à 10, dans laquelle les particules du sel inorganique comestible sont du carbonate de calcium précipité.

12. Composition comprenant l'émulsion selon l'une quelconque des revendications 1 à 11, où la composition est une composition alimentaire ; une boisson ; un agent d'amélioration de boisson ; une composition cosmétique ; une composition pharmaceutique ; une formule nutritionnelle ; ou une formulation d'alimentation par sonde.

13. Composition selon la revendication 12, dans laquelle la composition alimentaire est un produit laitier ; une crème glacée ; une sauce ; une soupe ; un produit à tartiner ; un dessert ; un produit de confiserie ; un produit de boulangerie ; une sauce pour salade ; ou un produit alimentaire pour animal de compagnie.

14. Composition selon la revendication 12 ou la revendication 13, où la composition alimentaire contient une quantité de sodium inférieure ou égale à 140 mg pour 100 g.

15. Procédé de fabrication d'une émulsion double comprenant les étapes consistant à
- former une dispersion aqueuse de particules d'un sel inorganique comestible comportant de 0,5 à 20 % en poids d'acides gras revêtus ou adsorbés sur leur surface, dans lequel le sel inorganique comestible présente une solubilité dans l'eau inférieure à 1 g pour 100 ml à 20 °C et dans lequel au moins 80 % en poids des acides gras sont des acides gras en C6 à C12
- former une dispersion d'huile de particules d'un sel inorganique comestible comportant de 0,5 à 20 % en poids d'acides gras revêtus ou adsorbés sur leur surface, dans lequel le sel inorganique comestible présente une solubilité dans l'eau inférieure à 1 g pour 100 ml à 20 °C et dans lequel au moins 80 % en poids des acides gras sont des acides gras en C16 à C22
- et soit
émulsionner une phase huileuse dans ladite dispersion aqueuse pour former une émulsion huile-dans-l'eau et émulsionner l'émulsion huile-dans-l'eau dans ladite dispersion huileuse pour former une émulsion huile-dans-l'eau-dans-l'huile,
soit
émulsionner une phase aqueuse dans ladite dispersion d'huile pour former une émulsion eau-dans-l'huile et émulsionner l'émulsion eau-dans-l'huile dans ladite dispersion aqueuse pour former une émulsion eau-dans-l'huile-dans-l'eau.
